(19) Europäisches Patentamt
European Patent Office
Office européen des brevets

(11) **EP 2 455 756 A1**

(12) **EUROPEAN PATENT APPLICATION**

(43) Date of publication:
23.05.2012 Bulletin 2012/21

(51) Int Cl.:
*G01N 33/50* (2006.01)

(21) Application number: 10075742.6

(22) Date of filing: 17.11.2010

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Max-Planck-Gesellschaft zur Förderung
der Wissenschaften e.V.
80539 München (DE)

(72) Inventors:
• **Martinez, Nancy**
**44227 Dortmund (DE)**

• **Fuchs, Tobias**
**63776 Gunzenbach (DE)**
• **Hakkim, Abdul**
**Cambridge, MA 02139 (US)**
• **Waldmann, Herbert**
**44265 Dortmund (DE)**
• **Zychlinsky, Arturo**
**10117 Berlin (DE)**

(74) Representative: **Arth, Hans-Lothar**
**ABK Patent Attorneys**
**Jasminweg 9**
**14052 Berlin (DE)**

(54) **Cell-based screening assay for inhibitors of NET formation**

(57) The present invention is directed to a cell-based screening assay for identifying inhibitors of NET formation.

Figure 6

## Description

## Field of the invention

The present invention is directed to the development of a cell-based screening assay for identifying inhibitors of NET formation.

## Background of the invention

Neutrophils are innate immune cells that migrate to infected sites where they phagocytose, degranulate and form Neutrophil extracellular traps (NETs) to kill microbes. NETs are relevant in infections and are implicated in the pathogenesis of, sepsis, thrombosis, preeclampsia, cystic fibrosis and autoimmune diseases among others. They are formed through a distinctive cell death programme, called «netosis», which requires the activation of NADPH oxidase that produces reactive oxygen species (ROS). During NET formation the characteristic nuclear lobules of neutrophils disappear and the chromatin expands. Throughout this process the cytoplasmic membrane is intact. However, after three to four hours of stimulation the membrane breaks releasing chromatin decorated with antimicrobial proteins into the extracellular space. The most distinct morphological changes during this process are in the nucleus and differ in several parameters including area, perimeter and shape. The molecular mechanism underlying NET formation is poorly understood.

It has long been known that neutrophils use two strategies to kill invading pathogens: engulfment of microbes and secretion of anti-microbials. However, a novel third function was identified: formation of neutrophil extracellular traps (NETs), whereby neutrophils kill extracellular pathogens while minimizing damage to the host cells. Upon in vitro activation with the pharmacological agent phorbol myristate acetate (PMA), Interleukin 8, lipopolysaccharide (LPS), platelet activating factor (PAF), live bacteria or *Candida albicans* neutrophils release granule proteins and chromatin to form an extracellular fibril matrix known as NETs through an active process.

More recently, it has also been shown that not only bacteria but also pathogenic fungi such as *Candida albicans* induces neutrophils to form NETs that capture and kill C. *albicans* hyphal as well as yeast-form cells. NETs have also been documented in association with *Plasmodium falciparum* infections in children. NETs disarm pathogens with antimicrobial proteins such as neutrophil elastase and histones that are bound to the DNA. NETs provide for a high local concentration of antimicrobial components and bind, disarm, and kill microbes extracellularly independent of phagocytic uptake. In addition to their antimicrobial properties, NETs may serve as a physical barrier that prevents further spread of the pathogens. Furthermore, delivering the granule proteins into NETs may keep potentially injurious proteins like proteases from diffusing away and inducing damage in tissue adjacent to the site of inflammation.

However, NETs might also have a deleterious effect on the host, because the exposure of extracellular histone complexes could play a role during the development of autoimmune diseases like lupus erythematosus. NETs could also play a role in inflammatory diseases, as NETs could be identified in preeclampsia, a pregnancy related inflammatory disorder in which neutrophils are known to be activated. NETs also have been shown to be associated with the production of IgG antinuclear double stranded DNA antibodies in children infected with *Falciparum malaria.*

While it was originally proposed that NETs would be formed in tissues at a site of bacterial/yeast infection, NETs have also been shown to form within blood vessels during sepsis (specifically in the lung capillaries and liver sinusoids). Intra-vascular NET formation is tightly controlled and is regulated by platelets, which sense severe infection via platelet TLR4 and then bind to and activate neutrophils to form NETs. NETs formed in blood vessels can catch circulating bacteria as they pass through the vessels. These observations suggest that NETs might play an important role in the pathogenesis of infectious and inflammatory disorders.

Several bottlenecks complicate the search for compounds that can modulate the formation of NETs. These cells develop in the bone marrow and reach circulation when they are terminally differentiated where they have a half life of six hours. Due to their short half life and their developmental state, neutrophils cannot be transfected or transduced. Also, there are no available cell lines that faithfully mimic the characteristics of neutrophils. In chemical genetic analyses, small molecule modulators of protein function are employed to rapidly and conditionally perturb and subsequently analyze biological processes. Thus, there is a need for such (small molecule) inhibitors to study the molecular mechanisms and signalling pathways involved in NET formation.

Objective of the present invention is to provide a screening assay to identify inhibitors of NET formation. Such inhibitors of NET formation could be useful to study the molecular mechanisms and signalling pathways involved in NET formation. Furthermore, such inhibitors are potential medicaments for the treatment and/or prophylaxis of diseases wherein the NET formation is abnormally increased.

This objective is solved by the present cell-based screening assay for inhibitors of NET formation using purified human blood neutrophils. Further preferred embodiments of the present invention are disclosed in the dependent claims, the description, the figures and the examples.

Surprisingly it was found that a cell-based screening assay according to the present invention is highly useful for identifying inhibitors of NET formation.

## Description of the invention

The present invention is directed to a cell-based screening assay for inhibitors of NET formation using neutrophils.

The inventive method for identifying compounds that inhibit NET formation comprises or consists of the following steps:

a1) providing neutrophils,
b1) contacting the neutrophils with one or more activators of NET formation,
c1) contacting the neutrophils simultaneously, subsequently or beforehand to step b1) with one or more test compounds, and
d1) staining of the neutrophils with a cell viability marker
e1) determining cell viability of the neutrophils
f1) staining of the nuclear DNA of the neutrophils
g1) determining nuclear size and/or morphology of the nuclear DNA of the neutrophils
h1) identify test compounds that inhibit NET formation by combining the results of step e1) and g1)

or

d2) staining of the neutrophils with a cell viability marker together with staining of the nuclear DNA,
e2) determining cell viability of the neutrophils and determining nuclear size and/or morphology of the nuclear DNA of the neutrophils,
f2) identifying test compounds that inhibit NET formation by combining the results obtained in step e2).

The above method may comprise the further step c2:

c2) fixation of the neutrophils.

Preferably the steps e1) and e2) are characterized in that the neutrophils are discriminated in dead and viable cells.

The steps g1) and g2) are preferably characterized in that the nuclear size and/or morphology of the nuclear DNA of the neutrophils are discriminated in lobulated, delobulated, diffused NET and spread NET.

Preferably the steps h1) and h2) are characterized in that the cells are grouped in one of the following groups: viable cells with lobulated or delobulated nuclei, viable cells with diffused NET, dead cells with diffused NET or spread NET, and dead cells with lobulated or delobulated nuclei.

One preferred embodiment of the method of the present invention comprises or consists of the following steps:

a1) providing neutrophils,
b1) contacting the neutrophils with one or more activators of NET formation,
c1) contacting the neutrophils simultaneously, subsequently or beforehand to step b1) with one or more test compounds,
c2) fixation of the neutrophils,
d1) staining of the neutrophils with a cell viability marker
e1) determining cell viability of the neutrophil
f1) staining of the nuclear DNA of the neutrophil
g1) determining nuclear size and/or morphology of the nuclear DNA of the neutrophils
h1) identifying test compounds that inhibit NET formation by combining the results of steps e1) and g1).

The fundamental concept of the inventive method is based on the fact that during NET formation there are discrete changes in nuclear size and shape, which finally result in cell death. In the present invention four distinct nuclear morphologies were defined and data is provided showing that the expansion of the chromatin can be evaluated quantitatively and qualitatively and together with the evaluation of cell viability results in a method that is highly reproducible, stringently selective for morphological definitions and allows the automatic evaluation of the changes in nuclear morphology in NET formation and thus is suitable to assay for test compounds with potential inhibitory effects on NET formation at different stages of NET formation.

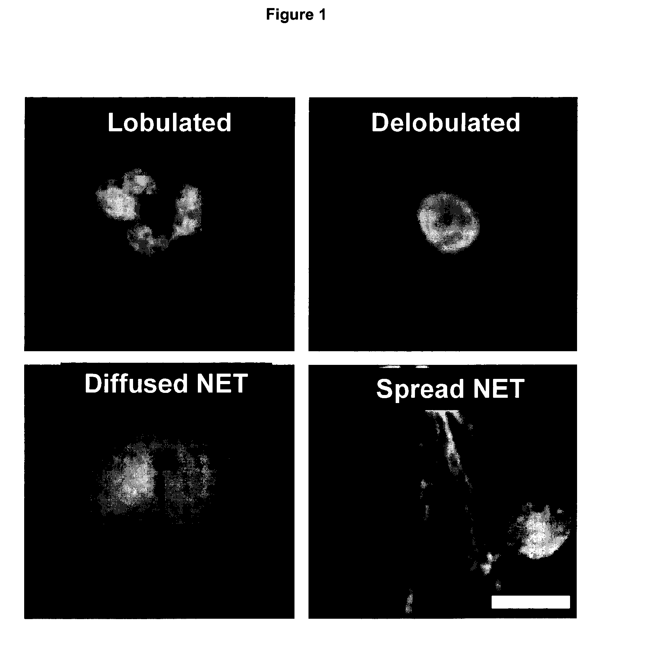

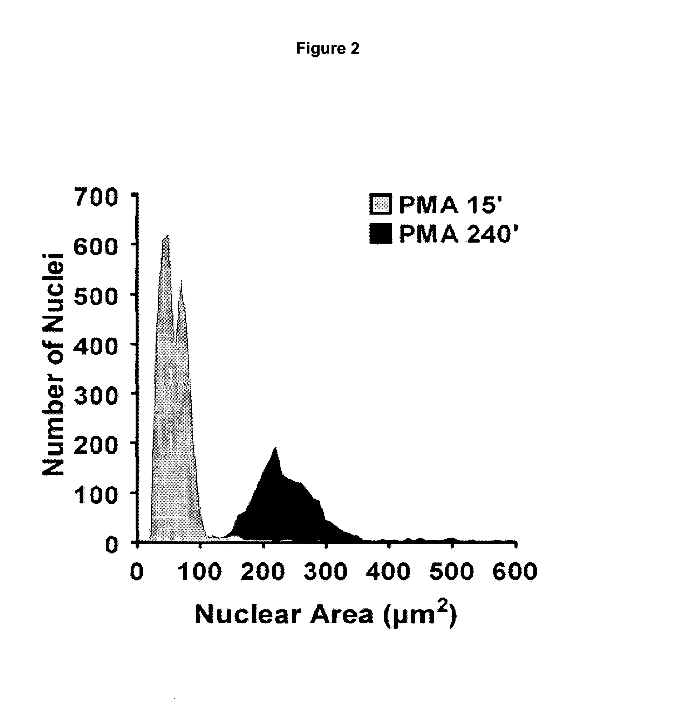

During NET formation there are discrete changes in nuclear size and shape. According to the invention four distinct nuclear morphologies were defined (Fig. 1) (i) lobulated, (ii) delobulated, (iii) diffused NETs and (iv) spread NETs. Neutrophils activated with PMA (40 nM) for 240 min had a larger nuclear area, which ranged between 150 and 350 $\mu m^2$ (mean:244$\mu m^2$; SD: 109$\mu m^2$; n: 2000; Fig. 2). In contrast, in cells activated for 15 min the nucleus had an area of less than 104 $\mu m^2$ (Fig. 2). The nuclear morphology, i.e. the morphology of the nuclear DNA was analyzed in more detail by plotting the perimeter vs. the Heywood circularity factor.

The Heywood circularity factor describes the ratio of the particle perimeter "p" to the perimeter of a circle having the same area A as the particle and is described by the following equation:

$$F_{\mathrm{H}} = \frac{p}{2\sqrt{\pi \cdot A}}$$

The Heywood circularity factor is useful to describe the shape of a certain particle or in this case the shape of the neutrophil nuclei.

The "particle perimeter" or "perimeter" as used herein refers to the distance around a given two-dimensional object, i.e. the distance around the stained nuclei as for example shown in Figs1.

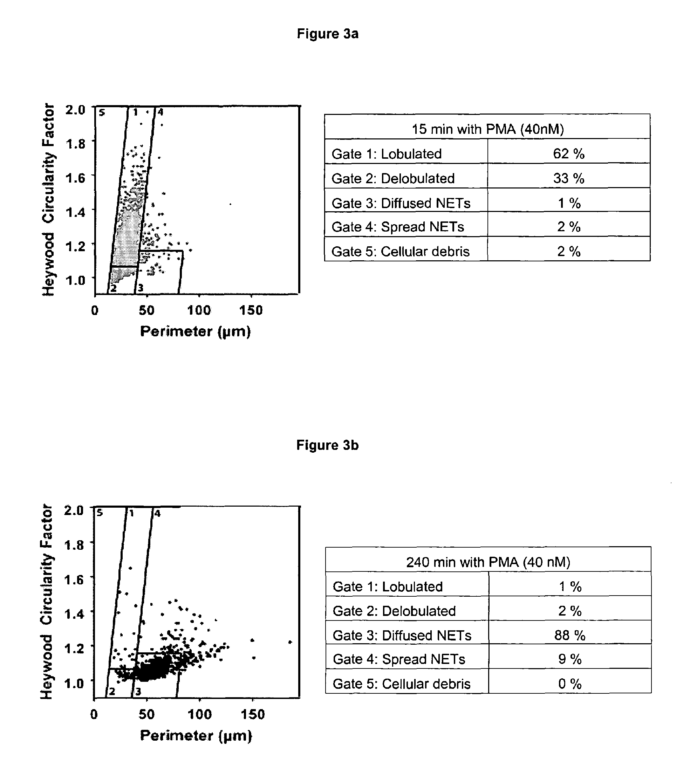

As can be seen in the dot plots of Figs. 3 (3a and 3b) the majority of nuclei of neutrophils stimulated for 15 min were either lobulated or delobulated and had a small perimeter and a high Heywood circularity factor (Fig. 3a). In contrast, most nuclei of neutrophils activated for 240 min, had diffused nuclei, or spread NETs that could be measured by their large perimeter and low Heywood circularity factor (Fig. 3b).

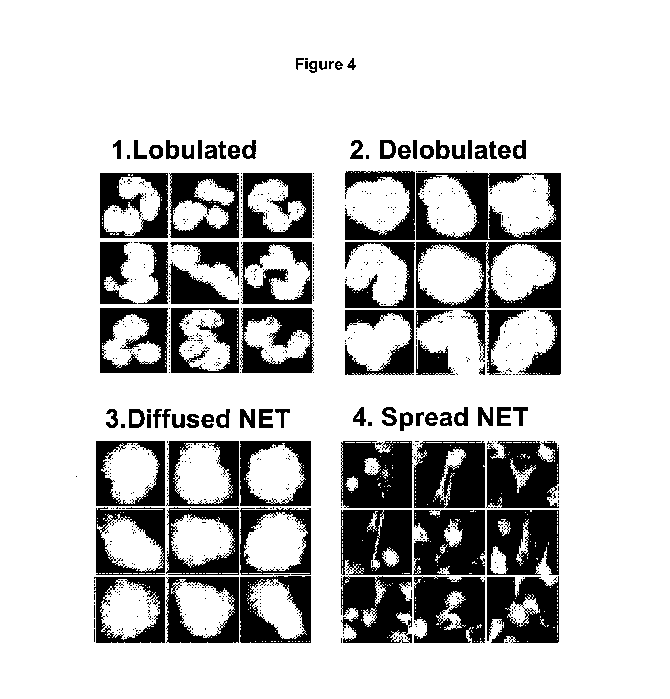

Fig. 4 shows representative images of the DNA staining of randomly picked cells within the center of each section of the dot plot shown in Figs. 3 (3a and 3b). These images show that the nuclear morphology as analyzed by plotting the perimeter vs. the Heywood circularity factor stringently discriminated in lobulated, delobulated, diffused NET and spread NET.

Together these data show that the expansion of the chromatin during NET formation can be quantified and qualitatively assessed leading to a highly reproducible and specific procedure, which in turn allows the automatic evaluation of the changes in nuclear morphology and thus is suitable to determine the inhibitory effect of potential NET inhibitory compounds at different stages of NET formation.

The term "lobulated" refers to the morphological appearance of the nuclei and describes a "segmented" or "overlapping" appearance of the nuclei as shown in Fig. 1 (top left view). The lobulated nuclei are preferably characterized by a perimeter between 5 to 30 $\mu$m and a Heywood circularity factor between 1.05 and 1.2.

The term "delobulated" refers to the morphological appearance of the nuclei and describes a more condensed appearance of the nuclei in comparison to the lobulated stage as shown in Fig. 1 (top right view). The delobulated nuclei are preferably characterized by a perimeter between 5 to 30 $\mu$m and a Heywood circularity factor between 1.0 and 1.05.

The term "diffused NET" refers to the morphological appearance of the nuclei and describes a gross enlarged nucleus during netosis with diffuse appearance as shown in Fig. 1 (bottom left view). The diffused nuclei are preferably characterized by a perimeter between 30 to 70 $\mu$m and a Heywood circularity factor between 1.05 and 1.25.

The term "spread NET" refers to the morphological appearance of the nuclei and describes the end stage of netosis where the chromatin is all spread out over a large area with a net like appearance as shown in Fig. 1 (bottom right view). The spread nuclei are preferably characterized by a perimeter between 50 to 150 $\mu$m and a Heywood circularity factor between 1.25 and 1.6.

According to the invention neutrophils and/or neutrophil-like cells of any origin can be used. In a preferred embodiment neutrophils of human origin are used. The isolation of such cells from human blood is well known in the art and can be accomplished for example by the method described by Eresso Aga et al., The Journal of Immunology, 2002, 169: 898-905. It is preferred if the percentage of neutrophils is at least 50% with regard to the total amount of cells used, more preferred 60%, even more preferred 70%, more preferably 80%, and most preferred at least 90%.

NET formation in neutrophils can be induced directly or indirectly by many physiologic and nonphysiologic activators of NET formation. This includes but is not restricted to phorbol-12-myristate-13-acetate (PMA), lipopolysaccharides (LPS), or platelet activating factor (PAF). According to the invention the neutrophils are contacted with one or more of such NET formation inducers for a time period sufficient to induce the formation of NET. This time period can vary depending on the origin of the neutrophils, the type of NET formation inducer used, the concentration used, the general condition of the neutrophil, the exact culture and assay conditions and other factors. The characteristic signs of NET formation are the disappearance of the nuclear lobules and the expansion of the chromatin. The most distinct morphological changes during this process are in the nucleus and differ in several parameters including area, perimeter and shape. Determining the necessary time period for NET formation can be accomplished, e.g. by comparing untreated neutrophils with PMA treated neutrophils. Typical time periods can range between 60 - 240 minutes but may vary for the reasons stated above.

The test compounds can be applied simultaneously, subsequently or beforehand to the induction of NET formation with the activator, whereas it is preferred to introduce the test compound and the NET formation activator simultaneously in a single step. However, in some embodiments it is preferred to pre-incubate the neutrophils with the test compounds so that they can develop their full inhibitory potential before the NET formation activator is added. In yet another embodiment the test compounds are introduced subsequently to the addition of the NET formation activator to evaluate compounds that are able to inhibit NET formation in later stages.

The term "test compound" as used herein refers to an agent comprising a compound, molecule, or complex that is being tested for its ability to inhibit NET formation. A test compound can be any agent including, but not restricted to, peptides, proteins, lipids, metals, nucleotides, nucleosides, small organic molecules, polyamines, and combinations and derivatives thereof. Complex mixtures of substances, such as extracts containing natural products, or the products of mixed combinatorial syntheses, can also be tested and the component that inhibits NET formation can be purified from the mixture in a subsequent step.

The fixation of the cells is conducted to ensure similar activation times and to prevent that NET formation can continue in the subsequent steps of the inventive method. Fixation is preferably carried out with paraformaldehyde, further preferred are fixative mediums that do not damage the cell membrane in any way. Nonetheless basically any fixative medium can be used to fix the cells even if they damage the cell membrane.

However, in certain embodiments it may be preferred to omit the fixation step. In these embodiments the method comprises or consists of the following steps:

a1) providing neutrophils,
b1) contacting the neutrophils with one or more activators of NET formation,
c1) contacting the neutrophils simultaneously, subsequently or beforehand to step b1) with one or more test compounds,
d1) staining of the neutrophils with a cell viability marker,
e1) determining cell viability of the neutrophils,
f1) staining of the nuclear DNA of the neutrophils,
g1) determining nuclear size and/or morphology of the nuclear DNA of the neutrophils,
h1) identifying test compounds that inhibit NET formation by combining the results of step e1) and g1)

Staining of nuclear DNA usually occurs with compounds that show high molar absorptivity with high extinction at visible wavelengths, very low intrinsic fluorescence, with low quantum yields when not bound to nucleic acids, large fluorescence enhancements upon binding to nucleic acids and moderate to very high affinity for nucleic acids, with little or no staining of other biopolymers. The compounds usually bind to the nucleic acid by intercalation, major groove binding, external binding, minor groove binding and/or bis-intercalation. Such dyes include but are not limited to ethidium bromide, propidium iodide, DAPI, Hoechst dyes, acridine orange, 7-AAD, LDS 751, hydroxystilbamidine and the various SYTOX® (Invitrogen) cyanine dyes.

In certain embodiments it is preferred to carry out the staining with the viability marker together with the staining of the nuclear DNA. In these embodiments the method comprises the following steps:

a1) providing neutrophils,
b1) contacting the neutrophils with one or more activators of NET formation,
c1) contacting the neutrophils simultaneously, subsequently or beforehand to step b1) with one or more test compounds,
c2) fixation of the neutrophils,
d2) staining of the neutrophils with a cell viability marker together with staining of the nuclear DNA,
e2) determining cell viability of the neutrophils and determining nuclear size and/or morphology of the nuclear DNA of the neutrophils,
f2) identify test compounds that inhibit NET formation by combining the results obtained in step e2)

According to the invention any type of cell viability assay can be used. Cell viability can be measured in a plurality of ways. One of the most basic indicators of viability is the metabolism of glucose and/or the active maintenance of membrane integrity. A healthy cell keeps certain materials on the inside and other materials on the outside of its membranes. Cell membrane integrity is commonly used to indicate cell viability. Loss of the protective cell membrane results in loss of cell structure, loss of critical intracellular contents, loss of essential ionic gradients and loss of electrical potential. The inevitable result of a major loss of membrane integrity is cell death. A common feature of loss of membrane integrity is the formation of pores which permit the passage of low molecular weight molecules (MW<2000 Daltons) in and out of the cytoplasm. This enhanced permeability has been the basis of many cell viability and cytotoxicity evaluations.

There is not an exact equivalence between an intact cell membrane and the term "viability" (technically defined as the ability of a cell to maintain its existence), it is common to refer to cells that have intact membranes as "viable" cells and cells where the membrane has been irreversibly disrupted as "dead" cells. There is, of course an intermediate condition where a cell that retains its membrane is in the process of "dying". A dying cell is not actually viable in that it cannot be cultured or reproduce. Dying cells are nevertheless often counted as living by common screening tests that rely on cell membrane integrity.

Thus, the term "cell viability" as used herein refers mostly to cells that have lost their membrane integrity as measured, e.g. by non-fluorescent Trypan Blue exclusion or fluorescent SYTOX® Green stain (Invitrogen). This mode of action is also often referred to as dye exclusion, whereby certain dyes can only enter the cell, after they have lost their membrane integrity. However, the meaning of "cell viability" as used herein is not restricted to the evaluation of membrane integrity and can also be directed to the measurement of activity of certain enzymes, protein expression or metabolic processes in general in the cell that might represent targets for the evaluation of cell viability. This could be assays such as the MTT-assay, which is based on the cleavage of the yellow tetrazolium salt MTT to purple formazan crystal by metabolic active cells.

According to the invention cell viability can be determined on a single cell basis or as a mean value of viable cells in comparison with a control. If the cell viability is determined on a single cell basis, determining cell viability comprises that a cell viability marker is used that can be analyzed by image acquiring, i.e. fluorescence microscopy or light microscopy. This way not only information about total cell viability is obtained but also which cells in specific are viable after treatment with the test compounds. In another embodiment the cell viability is determined not on a single cell basis, but as the mean value of all cells treated with the respective test compound. In this embodiment it is not necessary to use a cell viability marker that can be analyzed by image acquiring. This value can be expressed as a percent of viable cells in comparison with untreated control cells.

Based on the readouts of the cell viability marker and the readout of the detailed nuclear morphology, the inhibitory effect of test compounds on NET formation can be analyzed by grouping the cells in distinct groups. If the cell viability was determined on a single cell basis, then each cell can be grouped according to its viability in combination with its morphological status. This leads to the generation of the following distinct groups to identify test compounds that inhibit NET formation:

> 1. Viable cells with small nuclei (lobulated and delobulated),
> 2. Viable cells with large nuclei (diffused NET),
> 3. Dead cells with small nuclei (lobulated and delobulated),
> 4. Dead cells with large nuclei (diffused and spread NET)

The predominant occurrence of cells of group 1 after incubation with test compound points to an early inhibitory effect of the compound. On the other hand the predominant occurrence of cells of group 2 after incubation with the test compound is an indication for a delaying and/or an inhibitory effect in the later phases of netosis. If cells of group 3 are in the majority a cytotoxic effect of the test compound can be assumed, whereas the occurrence of cells of group 4 point to the fact that the corresponding test compound has no NET formation inhibitory potential.

In another embodiment the cell viability is not determined on a single cell basis but as a mean value for all cells treated with the respective test compound. The cells are then grouped according to their morphological status, namely lobulated, delobulated, diffused NETs and spread NETs but viability is only given as a mean value for all cells in comparison with e.g. an untreated control. The evaluation follows the above outlined procedure in that the predominant occurrence of viable cells with lobulated or delobulated nuclei after incubation with test compound points to an early inhibitory effect of the compound. Further that the predominant occurrence of viable cells with diffused NETs or spread NETs after incubation with the test compound is an indication for a delaying and/or an inhibitory effect in the later phases of netosis. If dead cells with lobulated or delobulated nuclei are in the majority a cytotoxic effect of the test compound can be assumed, whereas the occurrence of dead cells with diffused NETs or spread NETs point to the fact that the corresponding test compound has no NET formation inhibitory potential.

There are no fixed values when a test compound is considered to have NET formation inhibitory potential or not. This depends on a plurality of factors such as the origin of the neutrophils, the type of NET formation inducer, the test compound, the concentration, the general condition of the neutrophils, the exact culture and assay conditions and other factors. Evaluation of NET formation inhibitory potential must always be viewed with regard to an untreated control, whereas untreated refers only to the absence of test compound and not the NET formation inducer.

The fundamental concept of the inventive method can also be applied to a further embodiment of the invention. In this embodiment NET formation inducing compounds can be identified by a substantially similar procedure, by determining the cell viability and the discrete changes in nuclear size and shape but omitting the step of NET transformation activation with an activator. Consequently, in this embodiment the method comprises the following steps:

> a) providing neutrophils,
> b) contacting the neutrophils with one or more test compounds,
> c) fixation of the neutrophils,
> d) staining of the neutrophils with a cell viability marker,
> e) determining cell viability of the neutrophils,
> f) staining of the nuclear DNA of the neutrophils,
> g) determining nuclear size and/or morphology of the nuclear DNA of the neutrophils,
> h) identifying test compounds that activate NET formation by combining the results of step e) and g)

The cells are grouped according to their morphological status, namely lobulated, delobulated, diffused NETs and spread NETs. But the evaluation follows a different procedure in that the predominant occurrence of viable cells with lobulated or delobulated nuclei after incubation with test compound points to the fact that the corresponding test compound has no NET formation inducing potential. Further that the predominant occurrence of viable cells with diffused NETs or spread NETs after incubation with the test compound is an indication for a netosis inducing effect. If dead cells with lobulated or delobulated nuclei are in the majority a cytotoxic effect of the test compound can be assumed, whereas the occurrence of dead cells with diffused NETs or spread NETs point to a strong NET formation inducing activity of the test compound.

The above described advantageous embodiments and descriptions for a method of identifying NET formation inhibitors apply *mutatis mutandis* also for the method of identifying NET formation inducing compounds.

The inventive cell-based screening assay for inhibitors of NET formation is not restricted to the use of neutrophils but can also be applied to any type of cells capable of NET formation in general.

It is to be understood that, while the foregoing invention has been described in detail by way of illustration and example, numerous modifications, substitutions, and alterations are possible. Especially, the single steps of the inventive method and the different embodiments can be combined and/or performed in different order without departing from the spirit and scope of the invention as described in the following claims.

**Description of the figures**

Figure 1: Distinct nuclear morphologies during NET formation Neutrophils stained with SYTOX green, showing four distinct nuclear morphologies during NET formation: (i) lobulated, (ii) delobulated, (iii) diffused NETs and (iv) spread NETs. Bar = 10 μm

Figure 2: Nuclear area in neutrophils activated with PMA Measurement of nuclear area before and after activation with PMA

Figure 3: Dot plot nuclear morphology perimeter vs. the Heywood circularity factor after a) 15min PMA and b) 240 min PMA Dot plot of nuclei analyzed for perimeter vs. Heywood circularity factor.

Figure 4: Representative images of randomly picked cells within the center of each section of the dot plot shown in Figs. 3 Representative images of morphologies shown in Figs. 3 the images generated by an automated microscopy system and analyzed by image analysis software Scan ^R. (1) lobulated nuclei, (2) delobulated nuclei, (3) diffused NETs and (4) spread NETs.

Figure 5: Quantification of cell viability Different numbers of neutrophils per well were plated and activated with PMA for 240 min or incubated with the non ionic detergent NP-40.

Figure 6: Screening protocol and grouping: Flowchart showing one embodiment of the inventive method.

Examples

**Example 1:** Quantification of cell viability and nuclear morphology

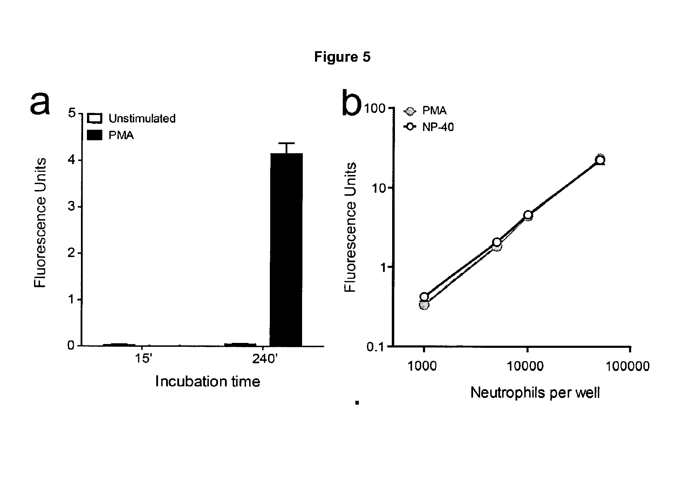

Neutrophils were activated with PMA (40 nM) for 15 min and 240 min, stained with the vital dye Sytox Green ($2\mu$M) and analyzed with a fluorometer. Only cells activated for 240 min were fluorescent, indicating cell death (Fig. 5a). Different numbers of neutrophils per well were plated and activated with PMA for 240 min or incubated with the non ionic detergent NP-40. This comparison indicated that most cells die after activation and that there is a linear correlation between fluorescence and cell number (Fig 5b).

For nuclear size and shape, neutrophils were activated with PMA for 240 min, fixed with PFA (2%), permeabilized with Triton X100 (0.1%) and stained with Sytox Green. The four distinct nuclear morphologies during the progression to NET formation are lobulated, delobulated or diffused NETs and spread NETs (Fig.1). Bar=10 μm

**Example 2:** Measurement of nuclear area using an automated microscopy system

Neutrophils were activated with PMA (40nM). Fifteen min after activation the nuclei were homogenous and smaller than 100 $\mu$m$^2$ in area while the nuclei of activated neutrophils were heteregenous and between 150 and more than 350 $\mu$m$^2$ (Figs. 3). Each dot in Figs. 3 (Dot plot of nuclei analyzed for perimeter vs. Heywood circularity factor) represents a nucleus. Gates were set to distinguish between lobulated, delobulated, diffused NETs and spread NETs.

In Fig. 3a fifteen min after activation, more than 95% of the nuclei were either lobulated or delobulated. Fig. 3b shows 240 min after activation more than 95% of the nuclei were either diffused or already made NETs. Fig. 4 shows representative images of morphologies shown in panels of Figs 3a/b. The images were generated by an automated microscopy system and analyzed by image analysis software Scan ^R.

**Example 3:** Neutrophil isolation and storage

Human neutrophils were isolated from blood of healthy donors by density gradient using the Polymorphprep™ system (Axis-Shield, Fisher Scientific, # AN1114683).

20 - 30 ml of blood are sufficient for a whole screen of approximately 1300 test compounds.
1. 20 ml venous blood was drawn using a 30 ml heparinized syringe (100 $\mu$l heparin in a 30 ml syringe).
2. 20 ml blood was slowly layered on top of 20 ml Polymorphprep™ in a 50 ml Falcon tube.
3. The tube was centrifuged at 512 $\times$ g (without brake) for 30 min at room temperature.
4. 5 ml plasma and mononuclear cells were aspirated from the top layer (This plasma was used as medium supplement during the assays.
5. The neutrophil layer (5-10 ml) was collected into a 50 ml Falcon tube.
6. Sterile phosphate-buffered saline was added to a volume of 50 ml and centrifuged at 512 $\times$ g for 10 min.
7. The supernatant was removed and 5 ml of sterile molecular grade water added to lyse erythrocytes.
8. 45 ml of PBS were added and centrifuged at 512 $\times$ g for 10 min. (2x)
9. Supernatant was discarded and the neutrophils resuspended in 1000 $\mu$l PBS.

The study was approved by the ethics committee of the Charité Medical Centre, Berlin. If not stated otherwise, purified neutrophils were stored in complete medium (RPMI medium without phenol-red supplemented with 10mM Hepes, 10% fetal calf serum, 2 mM L-glutamine, 100 U / ml penicillin, 100 U / ml streptomycin). Cells were diluted at least 100-fold in RPMI medium (phenol red-free) supplemented with 10 mM Hepes. 5 × 105-106 neutrophils / ml were seeded into tissue culture plates. Incubations were performed at 37 °C in the presence of 5% $CO_2$.

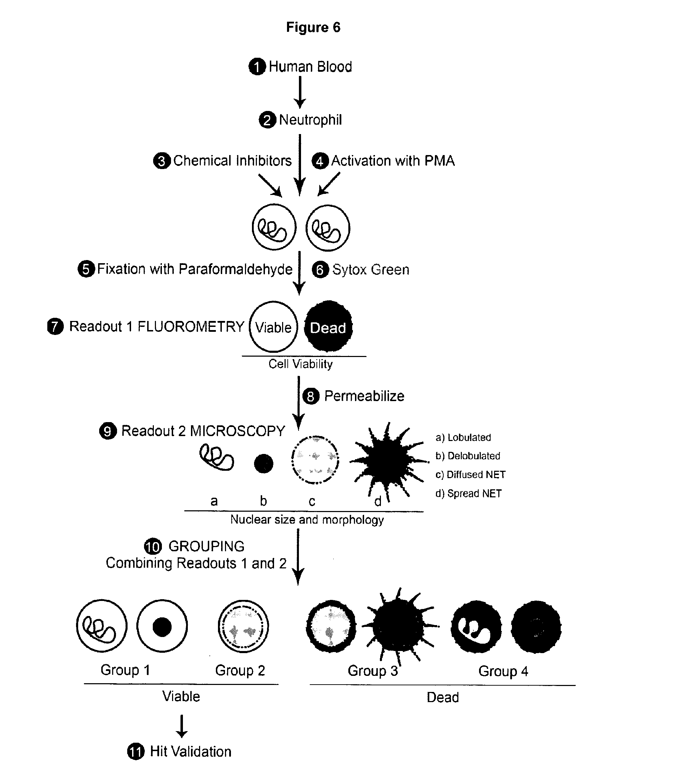

**Example 4:** Screening protocol and grouping according to Fig.6

The following steps (1-2) were performed manually. Human peripheral blood was drawn (1) and neutrophils were isolated using a density gradient (2). Small molecules were arrayed into 384 well plates by a pipetting robot (3). Ten thousand neutrophils were seeded to each well (2). Cells were activated with PMA (40 nM) for 240 min (4). Neutrophils were fixed with PFA (2%) and stained with the cell-impermeable DNA dye Sytox Green (5 and 6). At this step only dead neutrophils get stained. Fluorescence was determined to assess cell viability (7). Neutrophils were permeabilized to allow homogenous staining of all cells (8). Pictures of nuclear morphologies were taken by an automated microscopy system and classified into four distinctive morphologies (9). Based on readouts (step 7 and 9) hits were grouped (10). Viable cells with small nuclei (lobulated or rounded) in group 1 and with large nuclei in group 2. Non-inhibitors in group 3 and dead cells with small nuclei in group 4. Validation of the hits (11).

**Example 5:** Screen

Library compounds (small molecules (Sigma)) were arrayed by a pipetting robot (Zymark SciClone ALH 500 in conjunction with a Tecan EvoWare robot) in sterile 384, clear bottom, microtiter plates (MTPs) at a working concentration of 100 μM. Next 10,000 neutrophils were seeded per well and activated with 40 nM PMA. DMSO, the compound library solvent, was added to control samples. For cell death analysis, 2 μM Sytox Green together with 2% PFA was added after 4h of incubation and Sytox fluorescence was determined by an Ascent Fluoroskan MTP reader (Thermo Scientific) to assess cell viability. To analyze the nuclear area, cells were treated 0.01% triton X100, which permeabilizes the cells, allowing Sytox to stain viable neutrophils. The images of DNA stainings were analyzed using special software. The algorithm used in the image analysis software detects objects based on an intensity threshold for the fluorescent signal. A decrease in threshold leads to an increase in the detected object size. Therefore, the sensitivity to fluorescence was set very low to allow the software to compute multilobulated nucleus of naive neutrophils as a single particles. The images were acquired and analyzed with a Scan^R automated microscopy system (Olympus).

Following image acquisition, quantitative measurements from each image were performed based on the size of the nuclei and detailed morphology of NETs with parameters described above. For the detection of nuclei with the image analysis software an algorithm was applied based on an intensity threshold for the Sytox signal. The threshold for this screen was set to 200, with lowest intensities measured (background) around zero and a maximum of 4095. This relatively low threshold allows the detection of objects with low intensities, but also leads to an increased object size. Increasing the threshold would allow a more appropriate measurement of the actual size of the detected object, but would have the consequence that less bright objects could not be detected and that single lobules of the nucleus of naïve neutrophils would be detected as separate objects.

For the automated screening protocol pipetting steps were performed with the Zymark SciClone ALH 500 in conjunction with a Tecan EvoWare robot. The reaction volume was less than 20 μl, minimizing the expenditure of compounds. Initially, 10 μl of a neutrophil suspension were seeded in sterile 384 microtiter plates. The cells were allowed to settle for 10 min and the compound library was added at a working concentration of 100 μM in a volume of 0.2 μl. Then, 10 μl PMA at 50nM working concentration was added. DMSO, the compound library solvent, was added to control samples. To assess cell viability, 2 μM Sytox Green was added after 4h of incubation and Sytox fluorescence was determined by a Genios Pro MTP reader. Based on the cell viability as well as nuclear size and shape readouts were classified as described. The screen identified a couple of future candidate compounds for drug development programs aimed at prevention of NET formation and treatment of diseases associated with NETs.

**Claims**

1.   A method of identifying compounds that inhibit NET formation, wherein said method comprises the following steps:

a1) providing neutrophils,
b1) contacting the neutrophils with one or more activators of NET
formation,
c1) contacting the neutrophils simultaneously, subsequently or beforehand to step b1) with one or more test compounds,
and
d1) staining of the neutrophils with a cell viability marker,
e1) determining cell viability of the neutrophils,
f1) staining of the nuclear DNA of the neutrophils,
g1) determining nuclear size and/or morphology of the nuclear DNA of the

neutrophils

h1) identify test compounds that inhibit NET formation by combining

the results of step e1) and g1).

or

d2) staining of the neutrophils with a cell viability marker together with

staining of the nuclear DNA,

e2) determining cell viability of the neutrophils and determining nuclear

size and/or morphology of the nuclear DNA of the neutrophils,

f2) identifying test compounds that inhibit NET formation by combining the

results obtained in step e2).

2. The method according to claim 1 further comprising the step c2:

c2) fixation of the neutrophils.

3. The method according to claim 1 or 2, wherein the neutrophils are of human origin.

4. The method according to claims 1 - 3, wherein step e1) or step e2) is **characterized in that** the neutrophils are discriminated in dead and viable cells.

5. The method according to claims 1 - 4, wherein step g1) or step e2) is **characterized in that** the nuclear size and/or morphology of the nuclear DNA of the neutrophils is discriminated in lobulated, delobulated, diffused NET and spread NET.

6. The method according to claims 1 - 5, wherein step h1) or step f2) is **characterized in that** the cells are grouped in one of the following groups: viable cells with lobulated or delobulated nuclei, viable cells with diffused NET, dead cells with diffused NET or spread NET, and dead cells with lobulated or delobulated nuclei.

7. The method according to claims 1 - 6, wherein the discrimination of the neutrophils in lobulated, delobulated, diffused NET and spread NET is based on the analysis of the nuclei for perimeter ($\mu$m) vs. Heywood circularity factor.

8. A method of identifying compounds that induces NET formation, wherein said method comprises the following steps:

a) providing neutrophils,

b) contacting the neutrophils with one or more test compounds,

c) fixation of the neutrophils,

d) staining of the neutrophils with a cell viability marker,

e) determining cell viability of the neutrophils,

f) staining of the nuclear DNA of the neutrophils,

g) determining nuclear size and/or morphology of the nuclear DNA of the neutrophils,

h) identify test compounds that activate NET formation by combining the results of step e) and g).

9. The method according to claim 8, wherein the neutrophils are of human origin.

10. The method according to claim 8 or 9, wherein step e) is **characterized in that** the neutrophils are discriminated in dead and viable cells.

11. The method according to claims 8 - 10, wherein step g) is **characterized in that** the nuclear size and/or morphology of the nuclear DNA of the neutrophils is discriminated in lobulated, delobulated, diffused NET and spread NET.

12. The method according to claims 8 - 11, wherein step h) is **characterized in that** the cells are grouped in one of the following groups: viable cells with lobulated or delobulated nuclei, viable cells with diffused NET, dead cells with diffused NET or spread NET, and dead cells with lobulated or delobulated nuclei.

13. The method according to claims 8 - 12, wherein the discrimination of the neutrophils in lobulated, delobulated, diffused NET and spread NET is based on the analysis of the nuclear DNA for perimeter ($\mu$m) vs. Heywood circularity factor.

Figure 1

**Figure 2**

Figure 3a

| 15 min with PMA (40nM) | |
|---|---|
| Gate 1: Lobulated | 62 % |
| Gate 2: Delobulated | 33 % |
| Gate 3: Diffused NETs | 1 % |
| Gate 4: Spread NETs | 2 % |
| Gate 5: Cellular debris | 2 % |

Figure 3b

| 240 min with PMA (40 nM) | |
|---|---|
| Gate 1: Lobulated | 1 % |
| Gate 2: Delobulated | 2 % |
| Gate 3: Diffused NETs | 88 % |
| Gate 4: Spread NETs | 9 % |
| Gate 5: Cellular debris | 0 % |

Figure 4

## 1.Lobulated

## 2. Delobulated

## 3.Diffused NET

## 4. Spread NET

**Figure 5**

## Figure 6

**❶** Human Blood

**❷** Neutrophil

**❸** Chemical Inhibitors    **❹** Activation with PMA

**❺** Fixation with Paraformaldehyde    **❻** Sytox Green

**❼** Readout 1 FLUOROMETRY    (Viable)    (Dead)

Cell Viability

**❽** Permeabilize

**❾** Readout 2 MICROSCOPY

a) Lobulated
b) Delobulated
c) Diffused NET
d) Spread NET

a    b    c    d

Nuclear size and morphology

**❿** GROUPING
Combining Readouts 1 and 2

Group 1    Group 2    Group 3    Group 4

Viable    Dead

**⓫** Hit Validation

Europäisches
Patentamt

European
Patent Office

Office européen
des brevets

# EUROPEAN SEARCH REPORT

Application Number

EP 10 07 5742

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PALIC D ET AL: "Fish cast NETs: Neutrophil extracellular traps are released from fish neutrophils", DEVELOPMENTAL AND COMPARATIVE IMMUNOLOGY, PERGAMON PRESS, US, vol. 31, no. 8, 1 January 2007 (2007-01-01), pages 805-816, XP022705774, ISSN: 0145-305X, DOI: DOI:10.1016/J.DCI.2006.11.010 [retrieved on 2007-04-10] * the whole document * * page 809 * * figure 2 * | 1-13 | INV. G01N33/50 |
| X | FUCHS TOBIAS A ET AL: "Novel cell death program leads to neutrophil extracellular traps", JOURNAL OF CELL BIOLOGY, vol. 176, no. 2, January 2007 (2007-01), pages 231-241, XP002626808, ISSN: 0021-9525 * the whole document * * figure 1 * * page 232 * | 1-13 | |
| X | YOUSEFI S ET AL: "Viable neutrophils release mitochondrial DNA to form neutrophil extracellular traps", CELL DEATH AND DIFFERENTIATION, vol. 16, no. 11, November 2009 (2009-11), pages 1438-1444, XP002626809, ISSN: 1350-9047 * the whole document * * figure 3a * | 1-13 | TECHNICAL FIELDS SEARCHED (IPC) G01N |

-/--

The present search report has been drawn up for all claims

| Place of search | Date of completion of the search | Examiner |
|---|---|---|
| The Hague | 8 March 2011 | Jenkins, Gareth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 07 5742

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIPPOLIS J D ET AL: "Neutrophil extracellular trap formation by bovine neutrophils is not inhibited by milk", VETERINARY IMMUNOLOGY AND IMMUNOPATHOLOGY, AMSTERDAM, NL, vol. 113, no. 1-2, 15 September 2006 (2006-09-15), pages 248-255, XP024998977, ISSN: 0165-2427, DOI: DOI:10.1016/J.VETIMM.2006.05.004 [retrieved on 2006-09-15] * the whole document * * figure 1 * | 1-13 | |

TECHNICAL FIELDS
SEARCHED      (IPC)

The present search report has been drawn up for all claims

| Place of search | Date of completion of the search | Examiner |
|---|---|---|
| The Hague | 8 March 2011 | Jenkins, Gareth |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

............................................................................
& : member of the same patent family, corresponding
  document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ERESSO AGA et al.** *The Journal of Immunology,* 2002, vol. 169, 898-905